(19) **Europäisches Patentamt / European Patent Office / Office européen des brevets**

(11) **EP 4 051 105 B1**

(12) **EUROPEAN PATENT SPECIFICATION**

(45) Date of publication and mention of the grant of the patent:
**26.06.2024  Bulletin 2024/26**

(21) Application number: **20811721.8**

(22) Date of filing: **28.10.2020**

(51) International Patent Classification (IPC):
**A61B 5/103** (2006.01)

(52) Cooperative Patent Classification (CPC):
**A61B 5/1038;** A61B 2562/0247; A61B 2562/046

(86) International application number:
**PCT/IB2020/060077**

(87) International publication number:
**WO 2021/084427 (06.05.2021 Gazette 2021/18)**

(54) **METHOD FOR OPTIMISING THE ARRANGEMENT OF PRESSURE SENSORS AND DEVICE OBTAINED BY THIS METHOD**

VERFAHREN ZUR OPTIMIERUNG DER ANORDNUNG VON DRUCKSENSOREN UND NACH DIESEM VERFAHREN HERGESTELLTE VORRICHTUNG

PROCÉDÉ D'OPTIMISATION DE L'AGENCEMENT DE CAPTEURS DE PRESSION ET DISPOSITIF OBTENU PAR CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2019  IT 201900019902**

(43) Date of publication of application:
**07.09.2022  Bulletin 2022/36**

(73) Proprietor: **Scuola Superiore di Studi Universitari e di Perfezionamento Sant'Anna
56127 Pisa (IT)**

(72) Inventors:
  • **MARTINI, Elena**
    **50143 Firenze (IT)**
  • **BALDONI, Andrea**
    **56025 Pontedera (PI) (IT)**
  • **VITIELLO, Nicola**
    **56025 Pontedera (PI) (IT)**
  • **CREA, Simona**
    **55100 Lucca (IT)**
  • **FIUMALBI, Tommaso**
    **56025 Pontedera (IT)**
  • **DELL'AGNELLO, Filippo**
    **56025 Pontedera (IT)**

(74) Representative: **ABM Agenzia Brevetti & Marchi
Viale Giovanni Pisano, 31
56123 Pisa (IT)**

(56) References cited:
**US-A1- 2011 054 359**

• **SIMONA CREA ET AL: "A Wireless Flexible Sensorized Insole for Gait Analysis", SENSORS, vol. 14, no. 1, 9 January 2014 (2014-01-09), pages 1073-1093, XP055307223, DOI: 10.3390/s140101073**
• **LAETITIA CLAVERIE ET AL: "Discrete sensors distribution for accurate plantar pressure analyses", MEDICAL ENGINEERING & PHYSICS., vol. 38, no. 12, 1 December 2016 (2016-12-01), pages 1489-1494, XP055712334, GB ISSN: 1350-4533, DOI: 10.1016/j.medengphy.2016.09.021**
• **LIN SHU ET AL: "In-Shoe Plantar Pressure Measurement and Analysis System Based on Fabric Pressure Sensing Array", IEEE TRANSACTIONS ON INFORMATION TECHNOLOGY IN BIOMEDICINE, IEEE SERVICE CENTER, LOS ALAMITOS, CA, US, vol. 14, no. 3, 1 May 2010 (2010-05-01), pages 767-775, XP011345692, ISSN: 1089-7771, DOI: 10.1109/TITB.2009.2038904**

- TOKITA F ET AL: "PORTABLE INSTRUMENT FOR ACCURATE MEASUREMENT OF PLANTAR FORCE DISTRIBUTION DURING DYNAMIC ACTIVITIES", MEDICAL AND BIOLOGICAL ENGINEERING AND COMPUTING, SPRINGER, HEILDELBERG, DE, vol. 33, no. 4, 1 July 1995 (1995-07-01), pages 618-621, XP000516488, ISSN: 0140-0118

**Description**

Field of the invention

The present invention relates to the field of wearable robotics.

In particular, the invention relates to a sensorized device for detecting the phases of a user's footstep and a method for its optimization.

Description of the prior art

In the field of wearable robotics, it is necessary to know the details of the user's movement in real time, so that the robotic device can be operated in synergy with the person.

In particular, for walking assistance, and more generally for all the locomotor activities of daily life, it is important to monitor, through sensors, some characteristics of the person's movement so that the control algorithms of the robotic device can estimate what is the type of movement performed (walking, climbing stairs, being stationary in place, etc...) and how it is evolving (for example, during the walk, the phases in which the leg is lifted from the ground or in contact with the ground). Each movement and each of its sub-phases are in fact different from each other and therefore require different control actions on the behaviour of the robotic device. This process of so-called "decoding and segmentation of the movement" by the robot must be carried out in a safe, intuitive and minimally invasive way for the person.

Therefore, it is necessary to use sensors capable of providing the necessary information directly from the natural movement of the person, that is, without requiring the person to perform specific gestures to activate or modify the behaviour of the robot, integrating these sensors into clothing that is easy to wear and high comfort for the person.

In this context, plantar pressure detection technologies based on sensorized insoles/shoes can lend themselves to decoding the user's movement as well as the segmentation into phases of the movement performed, thanks to the real-time estimation of some biomechanical variables, such as the force exerted by the ground on the foot, or ground reaction force (GRF), and the center of pressures (CoP), i.e. its instantaneous point of application. Starting from these variables it is possible to trace some of the main biomechanical events of the gait cycle (e.g. "heel strike", "foot flat", "toe off") and its temporal determinants (support/oscillation phases, single/double support times).

EP2747645B1 describes a sensorized insole designed to be introduced inside a shoe to detect the pressures developed by the sole of a user's foot. In particular, this insole comprises a plurality of optical pressure sensors, adjacent to each other and arranged over the entire surface of the plantar support.

However, the presence of sensors on the entire walkable surface, in addition to being very economically expensive, reduces the space available to accommodate any hardware solutions that improve the durability of the individual sensors, making the use of the insole unsafe. In fact, the breakage of one of the sensors can also substantially modify the calculation of the forces and the center of pressures, and consequently distort the decoding and segmentation of the movement, making it necessary to replace the insole. The document SIMONA CREA ET AL: "A Wireless Flexible Sensorized Insole for Gait Analysis", SENSORS, vol. 14, no. 1, 9 January 2014 (2014-01-09), pages 1073-1093 represents relevant prior art and discloses a insole comprising 64 pressure sensors.

Summary of the invention

It is therefore a feature of the present invention to provide a method for the optimized arrangement of pressure sensors in a sensorized device arranged to detect biomechanical events characterizing the path and the phases of the footstep of a user, which allows decoding the movement of the user.

It is also a feature of the present invention to provide such a method that provides a device for detecting the pressures generated by the footstep of a user with a precision comparable to an insole having sensors over the entire surface, but requiring a smaller number of sensors.

It is still a feature of the present invention to provide such a method that allows to identify the spatial arrangement of sensors that requires the smallest number of sensors, while ensuring accurate decoding of the user's movement and detection of biomechanical events characterizing the path and its phases.

These and other objects are achieved by a method for the optimized arrangement of pressure sensors according to claims from 1 to 12.

It is also a feature of the present invention to provide a sensorized device arranged to detect the phases of the footstep according to claims 13 and 14.

It is a further feature of the present invention to provide a printed circuit arranged to be connected to the sensorized device, according to claims from 15 to 18.

Brief description of the drawings

Further characteristic and/or advantages of the present invention are more bright with the following description of an exemplary embodiment thereof, exemplifying but not limitative, with reference to the attached drawings in which:

- Fig. 1 diagrammatically shows the steps of a first embodiment of the method for the optimized arrangement of pressure sensors according to the present invention;
- Fig. 2 diagrammatically shows the steps of a second embodiment of the method for the optimized arrangement of pressure sensors according to the present invention;
- Fig. 3 diagrammatically shows the steps of a third embodiment of the method for the optimized arrangement of pressure sensors according to the present invention;
- Fig. 4 diagrammatically shows the steps of a fourth embodiment of the method for the optimized arrangement of pressure sensors according to the present invention;
- Fig. 5 shows a sensorized device arranged to detect the phases of the footstep of a user, according to the prior art;
- Fig. 6 shows a sensorized device optimised by the method for the optimized arrangement of pressure sensors according to the present invention;
- Fig. 7 shows a printed circuit connected to the optimized sensorized device, according to the present invention;
- Fig. 7A shows a detail of Fig. 7;
- Fig. 8 shows in detail a serpentine of the printed circuit of Fig. 7;
- Figs. 9A and 9B show the bending of a printed circuit of the prior art;
- Figs. 9C and 9D show the bending of a printed circuit with serpentine tracks according to the present invention;
- Fig. 10 shows a possible implementation of the optimised sensorized device on a robotic prosthesis;
- Fig. 11 shows a possible implementation of the optimised sensorized device on a robotic prosthesis comprising a cosmetic coating.

Description of a preferred exemplary embodiment

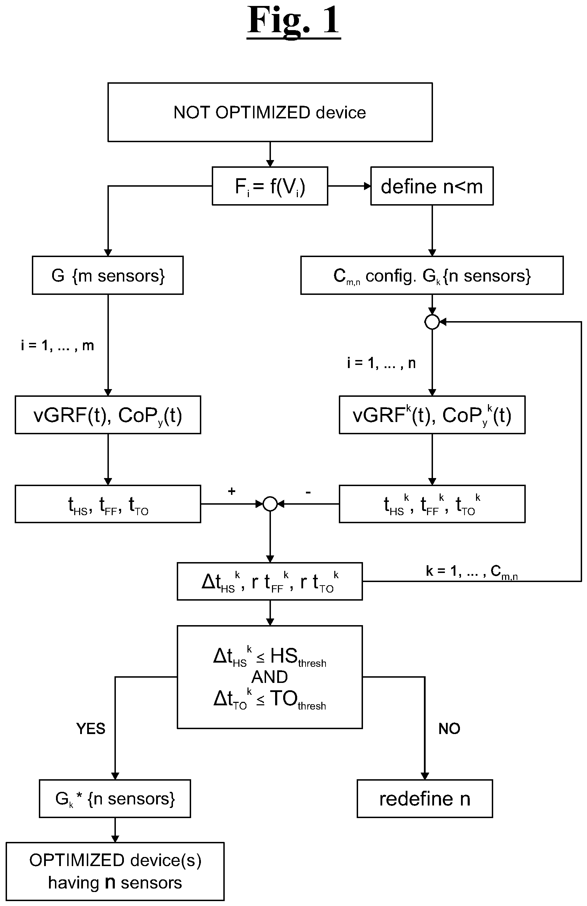

With reference to Figs. 1, 5 and 6, the method for the optimized arrangement of pressure sensors 100 in a sensorized device 10 arranged to detect the phases of the footstep of a user comprises a first step of prearranging a not optimised sensorized device 10'.

The not optimised sensorized device 10' comprises a group $G$ of $m$ pressure sensors 100, which are arranged in a working perimeter P and have coordinates $(x_i, y_i)$ with respect to a reference system $S(x, y)$ integral to said working perimeter P. Each pressure sensor 100 is identified with an index $i = 1, ... , m$ and is adapted to produce a corresponding output voltage $V_i$ in response to a pressure $p_i$ to which is subject.

The sensorized device 10' then comprises a control unit arranged to receive the output voltage $V_i$ from each pressure sensor (100) and to process a corresponding value of force $F_i = f(V_i)$.

The method then comprises a step of executing a working cycle using the not optimised sensorized device 10'. Such working cycle can be made by a user, wearing the sensorized device 10' and carrying out a normal walk. In particular, the working cycle can comprise one or more footsteps by the user, and each footstep comprises the phases of:

- early stance, i.e. the phase of resting the foot on the ground and accepting the load;
- propulsion, i.e. the phase in which the user carries the weight on the forefoot and pushes against the ground;
- flight, i.e. the phase in which the foot is lifted off the ground.

The three phases defined above are defined by the following three biomechanical events:

- "heel strike", i.e. the instant in which the foot hits the ground;
- "flat foot", i.e. the instant in which the plantar center of pressure is exactly in the middle of the anteroposterior axis and there is a load transition from the hind foot to the forefoot;
- "toe off", i.e. the instant in which the foot comes off the ground.

The execution of more than one footstep is preferable to increase the reliability of the data acquired by the control unit.

Subsequently, there is a step of processing the $m$ values of force $F_i(t)$, as a function of time t, starting from the output voltage $V_i$ obtained during the working cycle. Starting from the values of force $F_i(t)$, it is then carried out a calculation of the total force $vGRF(t)$ expressed on said not optimised sensorized device 10' using the equation:

$$vGRF(t) = \sum_{i=1}^{m} F_i(t) \quad F_i(t) = \begin{cases} f(V_i) \ V_i \leq V_{thresh} \\ 0 \ V_i > V_{thresh} \end{cases}$$

where $V_{thresh}$ is a predetermined threshold value.

In case that the voltage is lower than the threshold value, $V_i \leq V_{thresh}$, it is possible to determine the corresponding value of force $F_i(t) = f(V_i)$. On the contrary, the value of force is considered null.

By knowing the position of each pressure sensor 100 with respect to the reference system S(x,y), it is then possible to calculate the component $CoP_y$ (t) along the axis y of the centre of pressures, during all the working cycle, using the equation:

$$CoP_y \ (t) = \begin{cases} \dfrac{\sum_{i=1}^{m}(F_i \cdot y_i)}{\sum_{i=1}^{m}(F_i)} \ vGRF \geq vGRF_{thresh} \\ NaN \ vGRF < vGRF_{thresh} \end{cases}$$

where $vGRF_{thresh}$ is a predetermined threshold value.

If the value of force $vGRF$ is higher than the threshold value $vGRF_{thresh}$, the $CoP_y$ (t) has no value, i.e. $CoP_y$ (t) = $NaN$.

Then follows a step in which the component along the axis y of the geometric centre of the group for sensors 100 is calculated, using the equation:

$$CG_y = \frac{y_{max} + y_{min}}{2}$$

where $y_{max}$ and $y_{min}$ are, respectively, the maximum and minimum coordinate $y_i$ of the pressure sensors 100.

Once the centre of pressure $CoP_y$ (t) throughout the working cycle and the geometric centre $CG_y$ have been calculated, it is possible to define the transition instants between the various phases of the footstep.

In particular:

- when $CoP_y$ (t) passes from a $NaN$ value to a defined value, there is the starting instant of the phase of early stance $t_{HS}$, i.e. the "heel strike" instant;
- when $CoP_y$ (t) = $CG_y$, there is the starting instant of the propulsion phase $t_{FF}$, i.e. the "flat foot" instant;
- when $CoP_y$ (t) passes from a defined value to a $NaN$ value, there is the starting instant of the flight phase $t_{TO}$, i.e. the "toe off" instant.

Simultaneously or subsequently to the aforementioned steps, the method provides a step of defining the number $n < m$ of pressure sensors 100 desired in the sensorized device 10, once optimized.

Having defined the number $n$, a step is provided for identifying all the possible simple combinations of $n$ pressure sensors 100 in the group $G$ of $m$ sensors. Therefore, $C_{n,m}$ configurations $G_k$ are defined, with $k$ = 1, ... , $C_{n,m}$ and wherein:

$$C_{n,m} = \frac{m!}{(m-n)! \, n!}$$

Each group $G_k$ therefore corresponds to a possible final configuration of the $n$ sensors 100 in the optimised sensorized device 100 obtained as a result of the method.

For each configuration $G_k$ the steps carried out for the group $G$ of $m$ sensors are therefore repeated. In this way, it will then be possible to compare the results obtained for each $G_k$ configuration with the results obtained with the group of $m$ sensors and verify which arrangement of $n$ sensors 100 is more accurate in identifying the transition events between the real phases of the footstep.

Therefore, for each configuration $G_k$ a step is provided of computing:

- total force $vGRF^k(t)$;

- centre of pressures $CoP_y^k(t)$ ;

- initial instants of the various phases of the footstep $t_{HS}^k, t_{FF}^k, t_{TO}^k$ .

For each configuration $G_k$, the time distances are then calculated:

$$\Delta t_{HS}^k = \left| t_{HS}^k - t_{HS} \right|$$

$$\Delta t_{FF}^k = \left| t_{FF}^k - t_{FF} \right|$$

$$\Delta t_{TO}^k = \left| t_{TO}^k - t_{TO} \right|$$

The aforementioned time distances are a measure of how accurate each $G_k$ configuration is in defining the phases of the footstep of a user. Therefore, it's possible to proceed to the selection of the configurations $G_k^*$ for which are valid the conditions $\Delta t_{HS}^k \leq HS_{thresh}$ and $\Delta t_{TO}^k \leq TO_{thresh}$ , where $HS_{thresh}$ and $TO_{thresh}$ are predetermined thresholds of time distance.

The configurations $G_k^*$ are therefore the possible arrangements of the $n$ sensors 100 in the optimized sensorized device 10 which allow to carry out a recognition of the biomechanical events relevant for the definition of the phases of the movement performed, comparable to the non-optimized sensorized device 10', but with a small number of sensors 100 used.

In case that there are no configurations $G_k^*$ that meet the predetermined criteria, it is possible to change the number of sensors $n$ and/or change the threshold values $HS_{thresh}$ and $TO_{thresh}$.

In case, instead, that there is more than one configuration $G_k^*$ that meet the predetermined criteria, it is possible to evaluate the most suitable one as the configuration with the slightest error:

$$err = \Delta t_{HS}^k + \Delta t_{TO}^k + \frac{\left| \Delta t_{HS}^k - \Delta t_{TO}^k \right|}{2}$$

This measure allows to take into account the sum of the time distances and how close the time distances are to each other in absolute value, in order to obtain a more homogeneous precision configuration in the segmentation of the phases of the footstep.

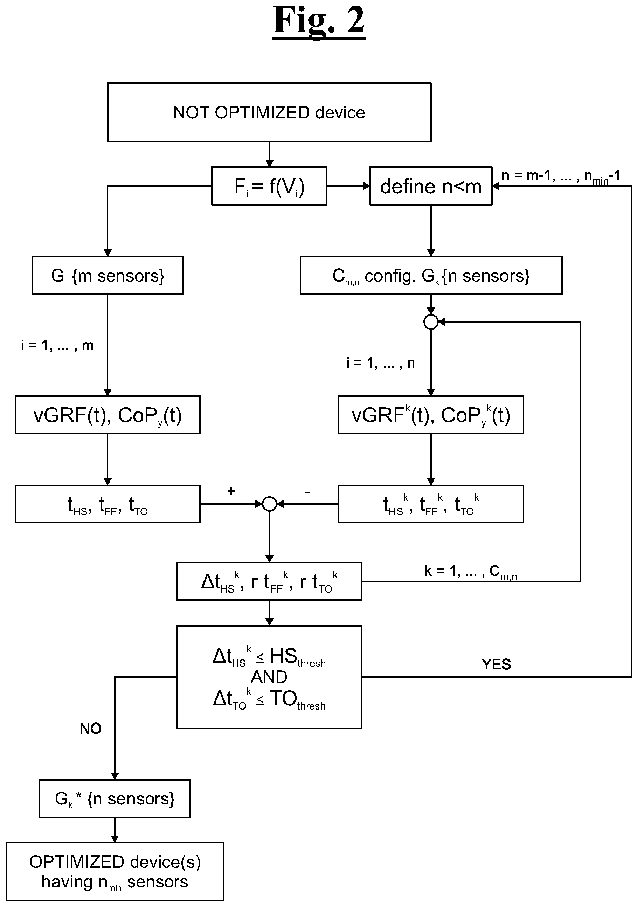

With reference also to Fig. 2, in a possible alternative of the method, the aim is to identify the configuration $G_k^*$ that satisfies the predetermined criteria on time distances, with the lowest number $n$ of pressure sensors 100. To do this, instead of fixing a predetermined value of $n$, we proceed with an iteration of the steps of the method, reducing the value of $n$ at each iteration. For example, the value of $n$ can start from the value $m$ - 1 and go to progressively reduce at each iteration until it reaches a minimum value $n_{min}$. This value is the smallest value of $n$ for which it is possible to select a number greater than zero of configurations $G_k^*$ . Therefore, the iteration will proceed up to determine a value $n = n_{min}$ - 1 for which there are no configurations $G_k^*$ meeting the predetermined criteria.

This way, it is possible to determine the configuration $G_k^*$ that meets the predetermined criteria on the time distances with the least possible number of pressure sensors 100.

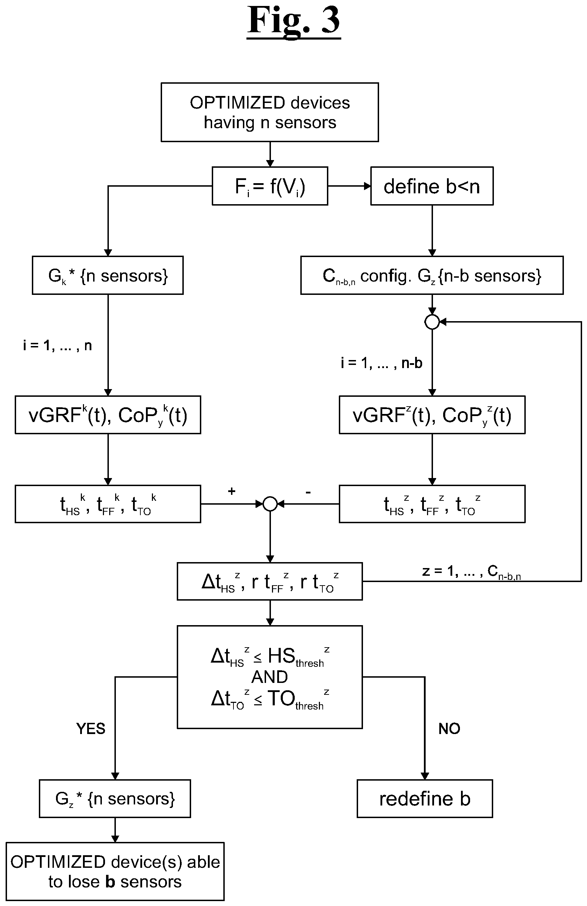

With reference to Fig. 3, in case that, at the end of the steps of the method according to Fig. 1, there are more configurations $G_k^*$ meeting the predetermined criteria, it is possible to select the best configuration, based on the number of pressure sensors 100 of which can bear the break, while maintaining the predetermined conditions over time distances.

In particular, downstream of the step of selecting the configurations $G_k^*$ , for each configuration $G_k^*$ the following steps are carried out:

- defining a number $b < n$ of pressure sensors 100 whose break or malfunction is to be simulated;
- defining a number $C_{n-b,n}$ of configurations $G_z$ containing $n - b$ pressure sensors 100 within the number $n$ of pressure sensors (100) arranged in the working perimeter P, with $z = 1, ..., C_{n-b,n}$, with:

$$C_{n-b,n} = \frac{(n-b)!}{b!\,n!}$$

- for each configuration $G_z$, computing the total force $vGRF^z(t)$ expressed on the sensorized device 10 by the equation:

$$vGRF^z(\mathrm{t}) = \sum_{i=1}^{n-b} F_i^z(\mathrm{t}) \quad F_i^z(\mathrm{t}) = \begin{cases} f(V_i) \; V_i \leq V_{thresh} \\ 0 \; V_i > V_{thresh} \end{cases}$$

- for each configuration $G_z$, computing the centre of pressures $CoPy(t)$ during the working cycle by the equation:

$$CoP_y^z(\mathrm{t}) = \begin{cases} \dfrac{\sum_{i=1}^{n-b}(F_i^z \cdot y_i)}{\sum_{i=1}^{n-b}(F_i^z)} \; vGRF^z \geq vGRF_{thresh} \\ NaN \; vGRF^z < vGRF_{thresh} \end{cases}$$

- for each configuration $G_z$, defining the instant $t_{HS}^z$ as instant wherein $CoP_y^z(\mathrm{t})$ passes from a $NaN$ value to a defined value, defining the instant $t_{FF}^z$ as instant wherein $CoP_y^z(\mathrm{t}) = CG_y$ , and defining the instant $t_{TO}^z$ as instant wherein $CoP_y^z(\mathrm{t})$ passes from a defined value to a $NaN$ value;
- for each configuration $G_z$, computing the time distances:

$$\Delta t_{HS}^z = \left| t_{HS}^z - t_{HS}^k \right|$$

$$\Delta t_{FF}^z = \left| t_{FF}^z - t_{FF}^k \right|$$

$$\Delta t_{TO}^z = \left| t_{TO}^z - t_{TO}^k \right|$$

- selecting configurations $G_z^*$ for which $\Delta t_{HS}^z \leq HS_{thresh}^z$ and $\Delta t_{TO}^z \leq TO_{thresh}^z$ , where $HS_{thresh}^z$ and $TO_{thresh}^z$ are predetermined thresholds of time distance.

This way, substantially, it is possible to determine, among all the configurations $G_k^*$ selected at the end of the method according to Fig. 1, only the configurations $G_z^*$ which can withstand the breakage of a number $b$ of sensors 100.

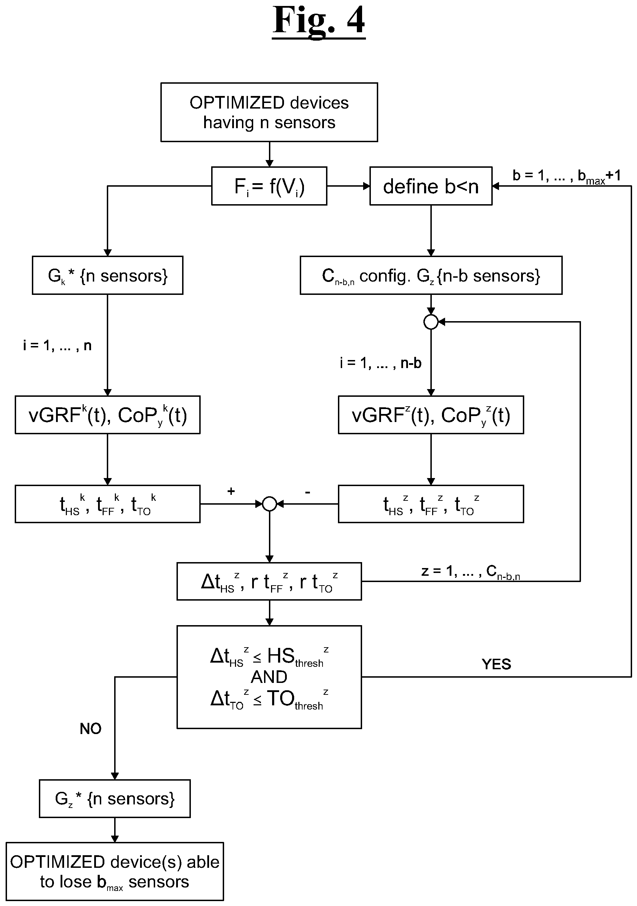

With reference to Fig. 4, in a possible alternative of the method, similarly to what has been seen for the method of Fig. 2, it is possible to iterate the steps of Fig. 3, with the aim of identifying the configuration $G_k$ capable of supporting the greatest number of breaks of the sensors 100.

In particular, at each iteration of the steps, $b$ assumes an increasing integer value set between 1 and $b_{max}$, where $b_{max}$ is maximum value of $b$ for which it is possible to select a number higher than zero of configurations $G_z^*$ .

Therefore, the iteration will proceed up to determine a value $b = b_{max} + 1$ for which there are no configurations $G_z^*$ that meet the predetermined criteria.

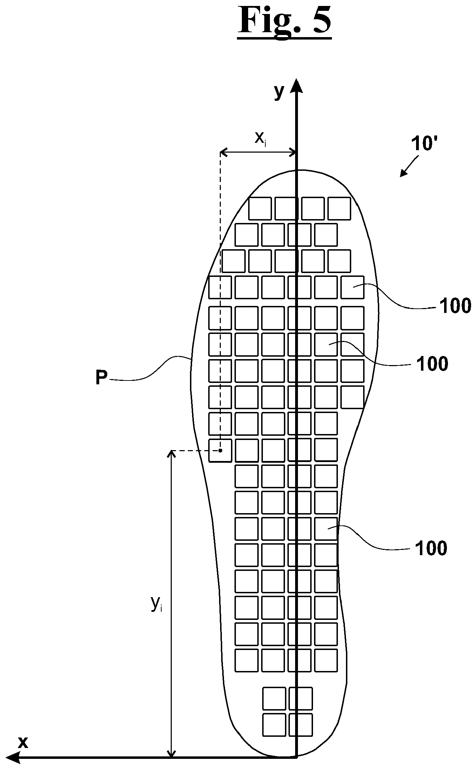

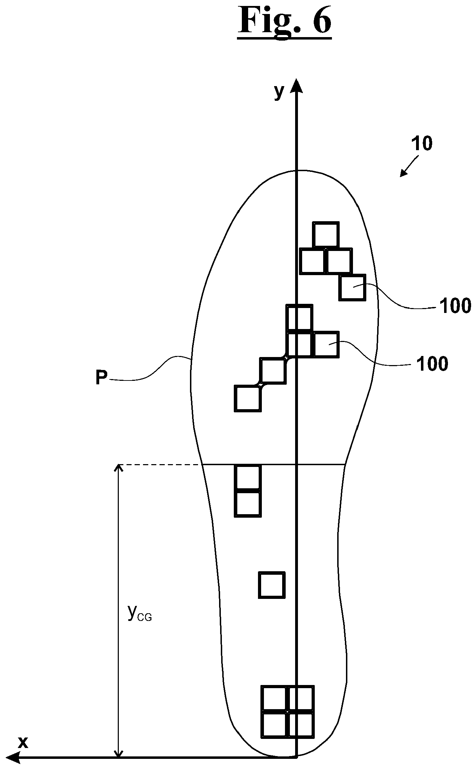

In Fig. 6 is shown a possible example of an optimised sensorized device 10. Starting from the number $m = 89$ of pressure sensors 100 present in the not optimised device 10' of Fig. 5, a possible configuration has been selected having a number $n = 16$ sensors 100, among which a number $n_{rear} = 7$ has coordinate $y_i < y_{CG}$ while a number $n_{front} = 9$ has coordinate $y_i > y_{CG}$.

In particular, the optimised sensorized device 10 has been obtained imposing, in the method according to the present invention, the following conditions:

- 

$$10N > vGRF_{thresh} > 20N$$

- $HS_{thresh} = 100ms$

- $TO_{thresh} = 100ms$.

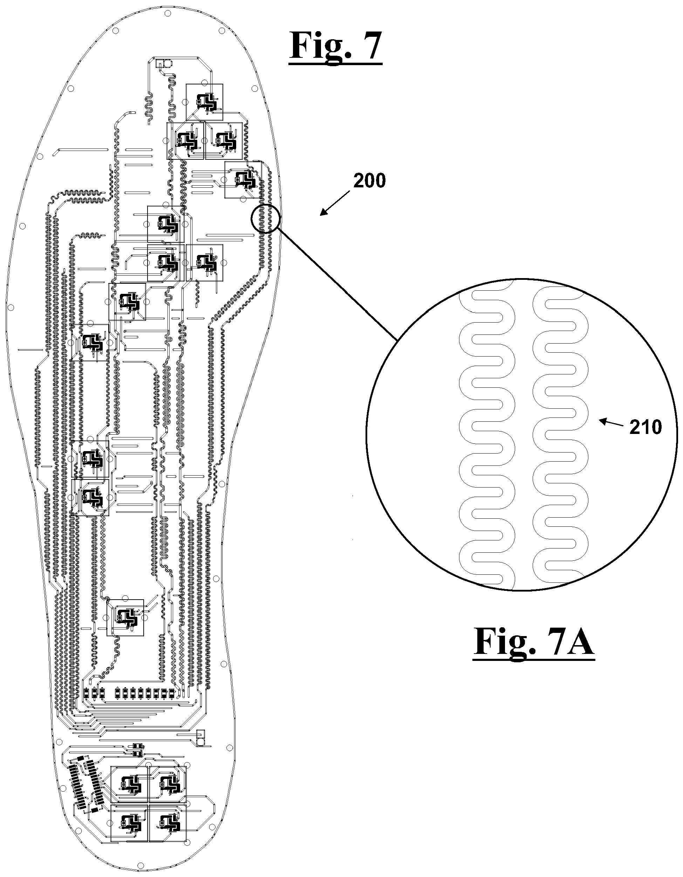

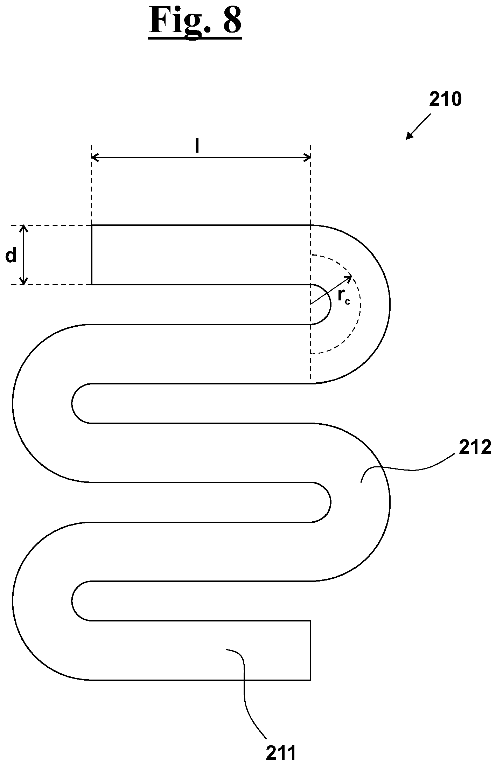

With reference to Figs. 7 and 8, the sensorized device 10 can also be connected to a printed circuit 200 comprising at least one track 210 having serpentine geometry and comprising at least two linear portions 211 of length $l$ and width $d$ and an arched connection portion 212 having radius of curvature $r_c$.

In particular, the following geometric conditions are present:

$$d < l < 5d$$

$$r_c \leq d$$

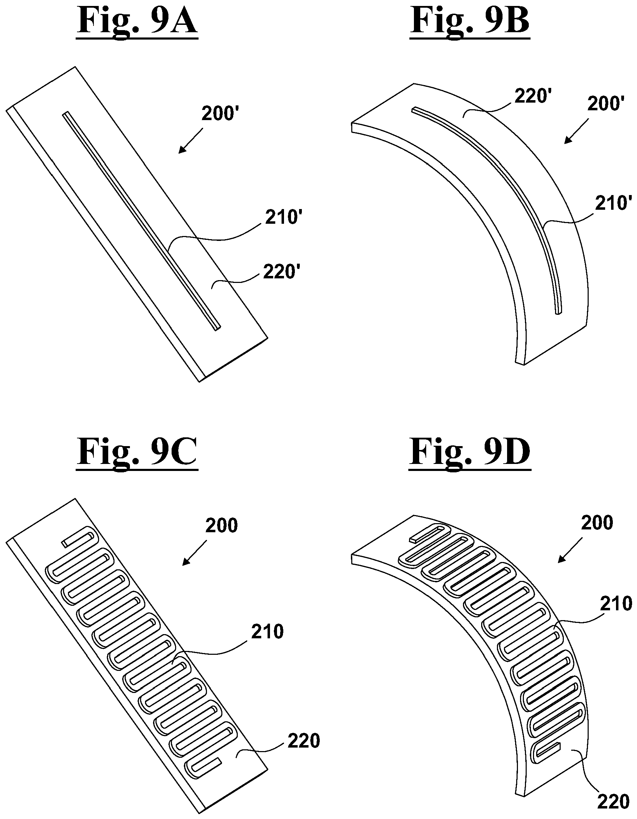

The particular serpentine structure of the conductive tracks makes it possible to greatly reduce the fatigue breakage due to the bending of the foot with respect to a printed circuit having straight conductive tracks. Figs. 9A, 9B, 9C and 9D show the different bending behaviour of a serpentine track with respect to a straight track of the prior art.

The embodiment of the sensorized device 10 of Fig. 6, comprising the printed circuit 200 of Fig. 7, is adapted for use as insole insertable, for example, inside a shoe.

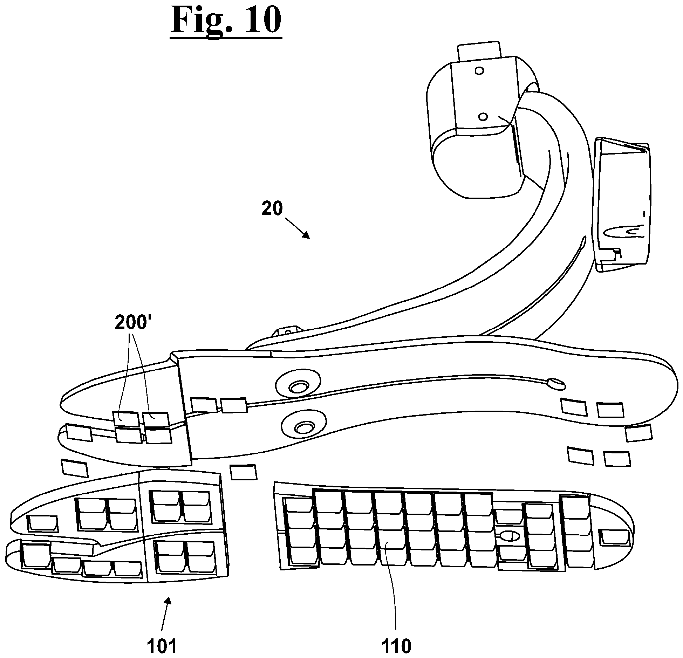

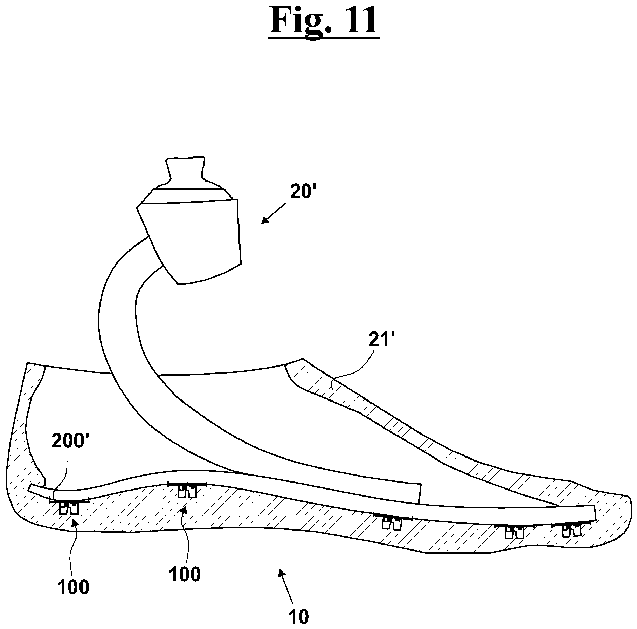

Alternatively, in Figs. 10 and 11 are shown two possible implementations of the optimised sensorized device 10 on two different robotic prostheses 20 and 20'. In both cases, unlike the embodiment of Figs. 6 and 7, it is not present a printed circuit 200 distributed over the entire surface insole, but a plurality of integrated circuits 200', of smaller size, arranged at the pressure sensors 100 selected by the method for optimization.

In particular, in Fig. 10 the sensorized device 10 is integrated into the prosthesis 20 by means of a silicone matrix 101 that comprises a plurality of housings 110 that, along with the printed circuits 200', constitute the pressure sensors 100. In this embodiment, the housings 110 are arranged over the entire the surface of the silicone matrix 101 to provide homogeneity of stiffness on the plant of the prosthesis when resting on the ground. However, only the housings 110 at which the printed circuits 200' are present are active sensors 100.

In Fig. 11 is shown a second possible implementation of the sensorized device 10 on a prosthesis. In particular, it is a robotic prosthesis 20' comprising a cosmetic coating 21'. In this solution, the sensors are inserted directly in the cosmetic coating 21' and the silicone matrix 101 is not present.

The foregoing description some exemplary specific embodiments will so fully reveal the invention according to the conceptual point of view, so that others, by applying current knowledge, will be able to modify and/or adapt in various applications the specific exemplary embodiments without further research and without parting from the invention, and, accordingly, it is meant that such adaptations and modifications will have to be considered as equivalent to the specific embodiments. The means and the materials to realise the different functions described herein could have a different nature without, for this reason, departing from the field of the invention, it is to be understood that the phraseology or terminology that is employed herein is for the purpose of description and not of limitation. The scope of the present invention is limited by the scope of the appended claims.

**Claims**

**1.** A method for the optimized arrangement of pressure sensors (100) within a sensorized device (10) arranged to detect biomechanical events characterizing the footstep of a user, said method comprising the steps of:

- prearranging a not optimised sensorized device (10') comprising:

  - a group $G$ of $m$ pressure sensors (100) arranged in a working perimeter P and having coordinates $(x_i, y_i)$ with respect to a reference system $S(x,y)$ integral to said working perimeter P, each pressure sensor (100) arranged to produce an output voltage $V_i$ in response to a pressure $p_i$ to which is subject, with $i = 1,...,m$;
  - a control unit arranged to receive said output voltage $V_i$ from each pressure sensor (100) and to process a corresponding value of force $F_i = f(V_i)$;

- executing a working cycle using said not optimised sensorized device (10'), each working cycle comprising at least one footstep comprising the phases of:

  - early stance;
  - propulsion;
  - flight;

- processing $m$ values of force $F_i(t)$, as a function of time t, starting from said values of output voltage $V_i$ acquired during said working cycle;
- computing the total force $vGRF(t)$ expressed on said not optimised sensorized device (10') by the equation:

$$vGRF(t) = \sum_{i=1}^{m} F_i(t) \quad F_i(t) = \begin{cases} f(V_i) & V_i \leq V_{thresh} \\ 0 & V_i > V_{thresh} \end{cases}$$

where $V_{thresh}$ is a predetermined threshold value;
- computing the centre of pressures $CoP_y(t)$ along an axis y of said reference system $S(x,y)$ during said working cycle by the equation:

$$CoP_y(t) = \begin{cases} \dfrac{\sum_{i=1}^{m}(F_i \cdot y_i)}{\sum_{i=1}^{m}(F_i)} & vGRF \geq vGRF_{thresh} \\ NaN & vGRF < vGRF_{thresh} \end{cases}$$

where $vGRF_{thresh}$ is a predetermined threshold value and $NaN$ is a not detectable value.
- computing the geometric centre $CG_y$ along an axis y of said reference system $S(x,y)$ by the equation:

$$CG_y = \frac{y_{max} + y_{min}}{2}$$

where $y_{max}$ and $y_{min}$ are, respectively, the maximum and minimum coordinate $y_i$ of said pressure sensors (100);
- defining the instant $t_{HS}$, i.e. the starting instant of said early stance phase, as the instant wherein $CoP_y(t)$ passes from a $NaN$ value to a defined value;
- defining the instant $t_{FF}$, i.e. the starting instant of said propulsion phase, as first instant wherein $CoP_y(t) \geq CG_y$;
- defining the instant $t_{TO}$, i.e. the starting instant of said flight phase, as instant wherein $CoP_y(t)$ passes from a defined value to a $NaN$ value;
- defining a number $n < m$ of pressure sensors (100) desired in said sensorized device (10);
- defining a number $C_{n,m}$ of configurations $G_k$ containing $n$ pressure sensors (100) in said group $G$ of $m$ of pressure sensors (100) in said working perimeter P, with $k = 1, ..., C_{n,m}$, with:

$$C_{n,m} = \frac{m!}{(m-n)!\, n!}$$

- for each configuration $G_k$, computing the total force $vGRF^k(t)$ expressed on said sensorized device (10) by the equation:

$$vGRF^k(\mathrm{t}) = \sum_{i=1}^{n} F_i^k(\mathrm{t}) \quad F_i^k(\mathrm{t}) = \begin{cases} f(V_i) \; V_i \leq V_{thresh} \\ 0 \; V_i > V_{thresh} \end{cases}$$

- for each configuration $G_k$, computing the centre of pressures $CoP_y^k(\mathrm{t})$ along an axis y of said reference system $S(x,y)$ during said working cycle by the equation:

$$CoP_y^k(\mathrm{t}) = \begin{cases} \dfrac{\sum_{i=1}^{n}(F_i^k \cdot y_i)}{\sum_{i=1}^{n}(F_i^k)} \quad vGRF^k \geq vGRF_{thresh} \\ \\ NaN \quad vGRF^k < vGRF_{thresh} \end{cases}$$

- for each configuration $G_k$, defining the instant $t_{HS}^k$ as instant wherein $CoP_y^k(\mathrm{t})$ passes from a *NaN* value to a defined value, defining the instant $t_{FF}^k$ as first instant wherein $CoP_y^k(\mathrm{t}) \geq CG_y$ , and defining the instant $t_{TO}^k$ as instant wherein $CoP_y^k(\mathrm{t})$ passes from a defined value to a *NaN* value;
- for each configuration $G_k$, computing the time distances:

$$\Delta t_{HS}^k = \left| t_{HS}^k - t_{HS} \right|$$

$$\Delta t_{FF}^k = \left| t_{FF}^k - t_{FF} \right|$$

$$\Delta t_{TO}^k = \left| t_{TO}^k - t_{TO} \right|$$

- selecting at least one configuration $G_k^*$ for which $\Delta t_{HS}^k \leq HS_{thresh}$ and $\Delta t_{TO}^k \leq TO_{thresh}$ , where $HS_{thresh}$ and $TO_{thresh}$ are predetermined thresholds of time distance;
- obtaining said sensorized device (10) comprising *n* pressure sensors (100) arranged according to one of said or each configuration $G_k^*$ .

2. The method for the optimized arrangement of pressure sensors (100), according to claim 1, wherein it is provided an iteration of the steps of:

- defining a number *n < m* of pressure sensors (100);
- defining a number $C_{n,m}$ of configurations $G_k$;
- for each configuration $G_k$:

  - computing the total force *vGRF^k*;
  - computing the centre of pressures $CoP_y^k(\mathrm{t})$ ;
  - defining the instants $t_{HS}^k$ , $t_{FF}^k$ , $t_{TO}^k$ ;
  - computing the time distances $\Delta t_{HS}^k = \left| t_{HS}^k - t_{HS} \right|$ , $\Delta t_{FF}^k = \left| t_{FF}^k - t_{FF} \right|$ and $\Delta t_{TO}^k = \left| t_{TO}^k - t_{TO} \right|$ ;

- selecting configurations $G_k^*$ for which $\Delta t_{HS}^k \leq HS_{thresh}$ and $\Delta t_{TO}^k \leq TO_{thresh}$ , where $HS_{thresh}$ and $TO_{thresh}$ are predetermined thresholds of time distance;

at each iteration of said steps, $n$ assuming a decreasing integer value set between $m$ - 1 and $n_{min}$, where $n_{min}$ is the minimum value of $n$ for which it is possible to select a number of configurations $G_k^*$ higher than zero.

3. The method for the optimized arrangement of pressure sensors (100), according to claims 1 or 2, wherein in said step of selecting they are selected configurations $G_k^*$ for which

$$\Delta t_{HS}^k \le HS_{thresh}, \quad \Delta t_{FF}^k \le FF_{thresh}, \quad \Delta t_{TO}^k \le TO_{thresh}$$

, where $FF_{thresh}$ is a predetermined threshold of time distance.

4. The method for the optimized arrangement of pressure sensors (100), according to claims 1 or 2, wherein it is also provided a step of defining a preferred configuration within said configurations $G_k^*$, said preferred configuration being the configuration having minimum the value:

$$err = \Delta t_{HS}^k + \Delta t_{TO}^k + \frac{\left|\Delta t_{HS}^k - \Delta t_{TO}^k\right|}{2}$$

5. The method for the optimized arrangement of pressure sensors (100), according to any of the previous claims, wherein downstream of said step of selecting said configurations $G_k^*$, for each configuration $G_k^*$ the following steps are provided:

   - defining a number $b < n$ of pressure sensors (100) whose break or malfunction is to be simulated;
   - defining a number $C_{n-b,n}$ of configurations $G_z$ containing $n$ - $b$ pressure sensors (100) within the number $n$ of pressure sensors (100) in said working perimeter $P$, with $z = 1, ..., C_{n-b,n}$, with:

$$C_{n-b,n} = \frac{(n-b)!}{b!\,n!}$$

   - for each configuration $G_z$, computing the total force $vGRF^z(t)$ expressed on said sensorized device (10) by the equation:

$$vGRF^z(t) = \sum_{i=1}^{n-b} F_i^z(t) \quad F_i^z(t) = \begin{cases} f(V_i) \ V_i \le V_{thresh} \\ 0 \ V_i > V_{thresh} \end{cases}$$

   - for each configuration $G_z$, computing the centre of pressures $CoP_y^z(t)$ along an axis y of said reference system $S(x,y)$ during said working cycle by the equation:

$$CoP_y^z(t) = \begin{cases} \dfrac{\sum_{i=1}^{n-b}(F_i^z \cdot y_i)}{\sum_{i=1}^{n-b}(F_i^z)} \ vGRF^z \ge vGRF_{thresh} \\ NaN \ vGRF^z < vGRF_{thresh} \end{cases}$$

   - for each configuration $G_z$, defining the instant $t_{HS}^z$ as instant wherein $CoP_y^z(t)$ passes from a $NaN$ value to a defined value, defining the instant $t_{FF}^z$ as instant wherein $CoP_y(t) = CG_y$, and defining the instant $t_{TO}^z$ as instant wherein $CoP_y^z(t)$ passes from a defined value to a $NaN$ value;
   - for each configuration $G_z$, computing the time distances:

$$\Delta t_{HS}^z = \left| t_{HS}^z - t_{HS}^k \right|$$

$$\Delta t_{FF}^z = \left| t_{FF}^z - t_{FF}^k \right|$$

$$\Delta t_{TO}^z = \left| t_{TO}^z - t_{TO}^k \right|$$

- selecting configurations $G_z^*$ for which $\Delta t_{HS}^z \leq HS_{thresh}^z$ and $\Delta t_{TO}^z \leq TO_{thresh}^z$, where $HS_{thresh}^z$ and $TO_{thresh}^z$ are predetermined thresholds of time distance.

**6.** The method for the optimized arrangement of pressure sensors (100), according to claim 5, wherein is provided an iteration of the steps of:

- defining the number $b < n$ of pressure sensors (100);
- defining a number $C_{n-b,n}$ of configurations $G_z$;
- for each configuration $G_z$:

  - computing the total force $vGRF^z(t)$;
  - computing the centre of pressures $CoP_y^z(t)$;
  - defining the instants $t_{HS}^z$, $t_{FF}^z$, $t_{TO}^z$;
  - computing the time distances $\Delta t_{HS}^z = \left| t_{HS}^z - t_{HS}^k \right|$, $\Delta t_{FF}^z = \left| t_{FF}^z - t_{FF}^k \right|$ and $\Delta t_{TO}^z = \left| t_{TO}^z - t_{TO}^k \right|$;

  - selecting configurations $G_z^*$ for which $\Delta t_{HS}^z \leq HS_{thresh}^z$ and $\Delta t_{TO}^z \leq TO_{thresh}^z$, where $HS_{thresh}^z$ and $TO_{thresh}^z$ are predetermined thresholds of time distance;

  at each iteration of said steps, $b$ assuming an increasing integer value set between 1 and $b_{max}$, where $b_{max}$ is the maximum value of $b$ for which it is possible to select a number higher than zero of configurations $G_z^*$.

**7.** The method for the optimized arrangement of pressure sensors (100), according to any of the previous claims, wherein, during said step of executing said working cycle, for each configuration $G_k$ there is a step of computing the total force $vGRF^k(t)$ and a step of computing the centre of pressures $CoP_y^k(t)$ by an external device, in order to compare the values of $vGRF^k(t)$ and $CoP_y^k(t)$ defined by said sensorized device (10) with the values defined by said external device.

**8.** The method for the optimized arrangement of pressure sensors (100), according to any of the previous claims, wherein said working cycle comprises a number N > 1 of footsteps and wherein all the defined values are calculated at each footstep and mediated to each other.

**9.** The method for the optimized arrangement of pressure sensors (100), according to any of the previous claims, wherein a step is also provided of connecting said sensorized device (10) to a printed circuit (200) comprising at least one conductive track (210) having serpentine geometry and comprising at least two linear portions (211) of length $l$ and width $d$ and an arched connection portion (212) having radius of curvature $r_c$.

**10.** The method for the optimized arrangement of pressure sensors (100), according to claim 9, wherein $d < l < 20d$.

**11.** The method for the optimized arrangement of pressure sensors (100), according to claim 9, wherein *d < l < 5d.*

**12.** The method for the optimized arrangement of pressure sensors (100), according to claim 9, wherein $r_c \leq d$.

**13.** A sensorized device (10) arranged to detect the phases of the footstep of a user comprising:

- a single group $G_k^*$ of pressure sensors (100), said single group $G_k^*$ consisting of *n* = 16 pressure sensors (100) arranged in a working perimeter P and having coordinates $(x_i, y_i)$ with respect to a reference system S(x,y), said working perimeter P defining a geometric centre $CG(x_{GG}, y_{GG})$ in said reference system S(x,y), each pressure sensor (100) arranged to produce an output voltage $V_i$ in response to a pressure $p_i$ to which is subject, with *i* = 1, ..., *n*;
- a control unit arranged to receive said output voltage $V_i$ from each pressure sensor (100) and to process a corresponding value of force $F_i = f(V_i)$;

wherein among said *n* = 16 pressure sensors (100):

- a number $n_{rear}$ of pressure sensors (100) has coordinate $y_i < y_{CG}$, with $5 < n_{rear} < 9$;
- a number $n_{front}$ of pressure sensors (100) has coordinate $y_i > y_{CG}$, with $7 < n_{front} < 11$.

**Patentansprüche**

**1.** Verfahren zur optimierten Anordnung von Drucksensoren (100) in einer mit Sensoren versehenen Vorrichtung (10), die so angeordnet ist, dass sie biomechanische Ereignisse erkennt, die den Schritt eines Fußes eines Benutzers charakterisieren, wobei das Verfahren die folgenden Schritte umfasst:

- Voranordnen einer nicht optimierten, mit Sensoren versehenen Vorrichtung (10'), die Folgendes umfasst:
- eine Gruppe *G* aus *m* Drucksensoren (100), die in einem Arbeitsumfang P angeordnet sind und Koordinaten $(x_i, y_i)$ in Bezug auf ein Referenzsystem *S(x,y)* aufweisen, das integraler Bestandteil des Arbeitsumfangs P ist, wobei jeder Drucksensor (100) so angeordnet ist, dass er eine Ausgangsspannung $V_i$ als Reaktion auf einen Druck $p_i$, dem er ausgesetzt ist, erzeugt, wobei *i* = 1,...,*m*;
- eine Steuereinheit, die so angeordnet ist, dass sie die Ausgangsspannung $V_i$ von jedem Drucksensor (100) empfängt und einen entsprechenden Kraftwert $F_i = f(V_i)$ verarbeitet;
- Ausführen eines Arbeitszyklus unter Verwendung der nicht optimierten, mit Sensoren versehenen Vorrichtung (10'), wobei jeder Arbeitszyklus mindestens einen Schritt eines Fußes umfasst, der die folgenden Phasen umfasst:

  - früher Stand;
  - Vorschub;
  - Schwung;
  - Verarbeiten von *m* Kraftwerten F;(t), als eine Funktion der Zeit t, ausgehend von den Werten der Ausgangsspannung $V_i$, die während des Arbeitszyklus erfasst wurden;
  - Berechnen der Gesamtkraft *vGRF(t),* die auf die nicht optimierte, mit Sensoren versehene Vorrichtung (10') ausgeübt wird, durch die folgende Gleichung:

$$vGRF(t) = \sum_{i=1}^{m} F_i(t) \quad F_i(t) = \begin{cases} f(V_i) \; V_i \leq V_{thresh} \\ 0 \; V_i > V_{thresh} \end{cases}$$

wobei $V_{thresh}$ ein vorbestimmter Schwellenwert ist;
- Berechnen des Druckmittelpunkts $CoP_y(t)$ entlang einer *y*-Achse des Referenzsystems *S(x,y)* während des Arbeitszyklus durch die folgende Gleichung:

$$CoP_y(t) = \begin{cases} \dfrac{\sum_{i=1}^{m}(F_i \cdot y_i)}{\sum_{i=1}^{m}(F_i)} & vGRF \geq vGRF_{thresh} \\ NaN & vGRF < vGRF_{thresh} \end{cases}$$

wobei $vGRF_{thresh}$ ein vorbestimmter Schwellenwert ist und *NaN* ein nicht erkennbarer Wert ist

- Berechnen des geometrischen Mittelpunktes $CG_y$ entlang einer *y*-Achse des Referenzsystems *S(x,y)* durch die folgende Gleichung:

$$CG_y = \frac{y_{max} + y_{min}}{2}$$

wobei $y_{max}$ und $y_{min}$ die maximale beziehungsweise die minimale Koordinate $y_i$ der Drucksensoren (100) sind;

- Definieren des Zeitpunkts $t_{HS}$, d. h. des Startzeitpunkts der frühen Standphase, als den Zeitpunkt, wobei $CoP_y(t)$ von einem *NaN*-Wert zu einem definierten Wert übergeht;
- Definieren des Zeitpunkts $t_{FF}$, d. h. des Startzeitpunkts der Vorschubphase, als ersten Zeitpunkt, wobei $CoP_y(t) \geq CG_y$;
- Definieren des Zeitpunkts $t_{TO}$, d. h. des Startzeitpunkts der Schwungphase, als Zeitpunkt, wobei $CoP_y(t)$ von einem definierten Wert zu einem *NaN*-Wert übergeht;
- Definieren einer Anzahl *n<m* von Drucksensoren (100), die in der mit Sensoren versehenen Vorrichtung (10) erwünscht sind;
- Definieren einer Anzahl $C_{n,m}$ von Konfigurationen $G_k$, die *n* Drucksensoren (100) enthalten, in der Gruppe G aus *m* Drucksensoren (100) in dem Arbeitsumfang P, mit $k = 1,...,C_{n,m}$, mit:

$$C_{n,m} = \frac{m!}{(m-n)!\,n!}$$

- für jede Konfiguration $G_k$, Berechnen der Gesamtkraft $vGRF^k(t)$, die auf die mit Sensoren versehene Vorrichtung (10) ausgeübt wird, durch die folgende Gleichung:

$$vGRF^k(t) = \sum_{i=1}^{n} F_i^k(t) \qquad F_i^k(t) = \begin{cases} f(V_i) & V_i \leq V_{thresh} \\ 0 & V_i > V_{thresh} \end{cases}$$

- für jede Konfiguration $G_k$, Berechnen des Druckmittelpunkts $CoP_y^k(t)$ entlang einer *y*-Achse des Referenzsystems *S(x,y)* während des Arbeitszyklus durch die folgende Gleichung:

$$CoP_y^k(t) = \begin{cases} \dfrac{\sum_{i=1}^{n}(F_i^k \cdot y_i)}{\sum_{i=1}^{n}(F_i^k)} & vGRF^k \geq vGRF_{thresh} \\ NaN & vGRF^k < vGRF_{thresh} \end{cases}$$

- für jede Konfiguration $G_k$, Definieren des Zeitpunkts $t_{HS}^k$ als Zeitpunkt, wobei $CoP_y^k(t)$ von einem *NaN*-Wert zu einem definierten Wert übergeht, Definieren des Zeitpunkts $t_{FF}^k$ als ersten Zeitpunkt, wobei $CoP_y^k(t) \geq CG_y$ und Definieren des Zeitpunkts $t_{TO}^k$ als Zeitpunkt, wobei $CoP_y^k(t)$ von einem definierten Wert zu einem *NaN*-Wert übergeht;
- für jede Konfiguration $G_k$, Berechnen der folgenden Zeitabstände:

$$\Delta t_{HS}^{k} = \left| t_{HS}^{k} - t_{HS} \right|$$

$$\Delta t_{FF}^{k} = \left| t_{FF}^{k} - t_{FF} \right|$$

$$\Delta t_{TO}^{k} = \left| t_{TO}^{k} - t_{TO} \right|$$

- Auswählen von mindestens einer Konfiguration $G_k^*$, für die $\Delta t_{HS}^{k} \leq HS_{thresh}$ und $\Delta t_{TO}^{k} \leq TO_{thresh}$, wobei $HS_{thresh}$ und $TO_{thresh}$ vorbestimmte Zeitabstandschwellenwerte sind;

- Herstellen der mit Sensoren versehenen Vorrichtung (10), die $n$ Drucksensoren (100) umfasst, die gemäß einer oder jeder Konfiguration $G_k^*$ angeordnet sind.

2. Verfahren zur optimierten Anordnung von Drucksensoren (100) gemäß Anspruch 1, wobei eine Iteration der folgenden Schritte bereitgestellt ist:

- Definieren einer Anzahl $n < m$ von Drucksensoren (100);
- Definieren einer Anzahl $C_{n,m}$ von Konfigurationen $G_k$;
- für jede Konfiguration $G_k$:
- Berechnen der Gesamtkraft $vGRF^k$;
- Berechnen des Druckmittelpunkts $CoP_y^k(t)$;
- Definieren der Zeitpunkte $t_{HS}^{k}$, $t_{FF}^{k}$, $t_{TO}^{k}$;
- Berechnen der Zeitabstände $\Delta t_{HS}^{k} = \left| t_{HS}^{k} - t_{HS} \right|$, $\Delta t_{FF}^{k} = \left| t_{FF}^{k} - t_{FF} \right|$ und $\Delta t_{TO}^{k} = \left| t_{TO}^{k} - t_{TO} \right|$;
- Auswählen von Konfigurationen $G_k^*$, für die $\Delta t_{HS}^{k} \leq HS_{thresh}$ und $\Delta t_{TO}^{k} \leq TO_{thresh}$, wobei $HS_{thresh}$ und $TO_{thresh}$ vorbestimmte Zeitabstandschwellenwerte sind;

wobei bei jeder Iteration der Schritte $n$ einen abnehmenden ganzzahligen Wert zwischen $m - 1$ und $n_{min}$ annimmt,

wobei $n_{min}$ der Mindestwert von $n$ ist, für den es möglich ist, eine Anzahl von Konfigurationen $G_k^*$ größer als Null auszuwählen.

3. Verfahren zur optimierten Anordnung von Drucksensoren (100) gemäß den Ansprüchen 1 oder 2, wobei im Schritt des Auswählens Konfigurationen $G_k^*$ ausgewählt werden, für die $\Delta t_{HS}^{k} \leq HS_{thresh}$, $\Delta t_{FF}^{k} \leq FF_{thresh}$, $\Delta t_{TO}^{k} \leq TO_{thresh}$, wobei $FF_{thresh}$ ein vorbestimmter Zeitabstandschwellenwert ist.

4. Verfahren zur optimierten Anordnung von Drucksensoren (100) gemäß den Ansprüchen 1 oder 2, wobei außerdem ein Schritt eines Definierens einer bevorzugten Konfiguration innerhalb der Konfigurationen $G_k^*$ bereitgestellt ist, wobei die bevorzugte Konfiguration die Konfiguration ist, die mindestens den folgenden Wert aufweist:

$$err = \Delta t_{HS}^{k} + \Delta t_{TO}^{k} + \frac{\left| \Delta t_{HS}^{k} - \Delta t_{TO}^{k} \right|}{2}.$$

5. Verfahren zur optimierten Anordnung von Drucksensoren (100) gemäß einem der vorhergehenden Ansprüche, wobei nachgelagert dem Schritt des Auswählens der Konfigurationen $G_k^*$ für jede Konfiguration $G_k^*$ die folgenden Schritte bereitgestellt sind:

- Definieren einer Anzahl $b<n$ von Drucksensoren (100), deren Bruch oder Fehlfunktion zu simulieren ist;
- Definieren einer Anzahl $C_{n\text{-}b,n}$ von Konfigurationen $G_z$, die $n - b$ Drucksensoren (100) enthalten, innerhalb der Anzahl $n$ von Drucksensoren (100) in dem Arbeitsumfang P, mit $z = 1, \dots, C_{n\text{-}b,n}$, mit:

$$C_{n-b,n} = \frac{(n-b)!}{b!\,n!}$$

- für jede Konfiguration $G_z$, Berechnen der Gesamtkraft $vGRF^z(t)$, die auf die mit Sensoren versehene Vorrichtung (10) ausgeübt wird, durch die folgende Gleichung:

$$vGRF^z(t) = \sum_{i=1}^{n-b} F_i^z(t) \quad F_i^z(t) = \begin{cases} f(V_i) & V_i \le V_{thresh} \\ 0 & V_i > V_{thresh} \end{cases}$$

- für jede Konfiguration $G_z$, Berechnen des Druckmittelpunkts $CoP_y^z(t)$ entlang einer $y$-Achse des Referenzsystems $S(x,y)$ während des Arbeitszyklus durch die folgende Gleichung:

$$CoP_y^z(t) = \begin{cases} \dfrac{\sum_{i=1}^{n-b}(F_i^z \cdot y_i)}{\sum_{i=1}^{n-b}(F_i^z)} & vGRF^z \ge vGRF_{thresh} \\ NaN & vGRF^z < vGRF_{thresh} \end{cases}$$

- für jede Konfiguration $G_z$, Definieren des Zeitpunkts $t_{HS}^z$ als Zeitpunkt, wobei $CoP_y^z(t)$ von einem $NaN$-Wert zu einem definierten Wert übergeht, Definieren des Zeitpunkts $t_{FF}^z$ als Zeitpunkt, wobei $CoP_y^z(t) = CG_y$ und Definieren des Zeitpunkts $t_{TO}^z$ als Zeitpunkt, wobei $CoP_y^z(t)$ von einem definierten Wert zu einem $NaN$-Wert übergeht;
- für jede Konfiguration $G_z$, Berechnen der folgenden Zeitabstände:

$$\Delta t_{HS}^z = \left| t_{HS}^z - t_{HS}^k \right|$$

$$\Delta t_{FF}^z = \left| t_{FF}^z - t_{FF}^k \right|$$

$$\Delta t_{TO}^z = \left| t_{TO}^z - t_{TO}^k \right|$$

- Auswählen von Konfigurationen $G_z^*$, für die $\Delta t_{HS}^z \le HS_{thresh}^z$ und $\Delta t_{TO}^z \le TO_{thresh}^z$, wobei $HS_{thresh}^z$ und $TO_{thresh}^z$ vorbestimmte Zeitabstandschwellenwerte sind.

6. Verfahren zur optimierten Anordnung von Drucksensoren (100) gemäß Anspruch 5, wobei eine Iteration der folgenden Schritte bereitgestellt ist:

- Definieren der Anzahl $b < n$ von Drucksensoren (100);
- Definieren einer Anzahl $C_{n\text{-}b,n}$ von Konfigurationen $G_z$;
- für jede Konfiguration $G_z$:
- Berechnen der Gesamtkraft $vGRF^z(t)$;
- Berechnen des Druckmittelpunkts $CoP_y^z(t)$;
- Definieren der Zeitpunkte $t_{HS}^z,\ t_{FF}^z,\ t_{TO}^z$;

- Berechnen der Zeitabstände $\Delta t_{HS}^z = \left| t_{HS}^z - t_{HS}^k \right|$ ,

$$\Delta t_{FF}^z = \left| t_{FF}^z - t_{FF}^k \right| \text{ und } \Delta t_{TO}^z = \left| t_{TO}^z - t_{TO}^k \right|;$$

- Auswählen von Konfigurationen $G_z^*$ , für die $\Delta t_{HS}^z \leq HS_{thresh}^z$ und $\Delta t_{TO}^z \leq TO_{thresh}^z$ , wobei $HS_{thresh}^z$ und $TO_{thresh}^z$ vorbestimmte Zeitabstandschwellenwerte sind;

wobei bei jeder Iteration der Schritte $b$ einen zunehmenden ganzzahligen Wert zwischen 1 und $b_{max}$ annimmt, wobei $b_{max}$ der Maximalwert von $b$ ist, für den es möglich ist, eine Anzahl von Konfigurationen $G_z^*$ größer als Null auszuwählen.

7. Verfahren zur optimierten Anordnung von Drucksensoren (100) gemäß einem der vorhergehenden Ansprüche, wobei während des Schritts des Ausführens des Arbeitszyklus für jede Konfiguration $G_k$ ein Schritt des Berechnens der Gesamtkraft $vGRF^k(t)$ und ein Schritt des Berechnens des Druckmittelpunkts $CoP_y^k(t)$ durch eine externe Vorrichtung erfolgt, um die Werte von $vGRF^k(t)$ und $CoP_y^k(t)$ , die durch die mit Sensoren versehene Vorrichtung (10) definiert werden, mit den Werten, die durch die externe Vorrichtung definiert werden, zu vergleichen.

8. Verfahren zur optimierten Anordnung von Drucksensoren (100) gemäß einem der vorhergehenden Ansprüche, wobei der Arbeitszyklus eine Anzahl $N > 1$ von Schritten eines Fußes umfasst und wobei bei jedem Schritt eines Fußes alle definierten Werte berechnet und zueinander in Beziehung gesetzt werden.

9. Verfahren zur optimierten Anordnung von Drucksensoren (100) gemäß einem der vorhergehenden Ansprüche, wobei außerdem ein Schritt eines Verbindens der mit Sensoren versehenen Vorrichtung (10) mit einer gedruckten Schaltung (200) bereitgestellt ist, die mindestens eine leitende Bahn (210) mit serpentinenförmiger Geometrie umfasst und mindestens zwei lineare Abschnitte (211) mit einer Länge $l$ und einer Breite $d$ und einem bogenförmigen Verbindungsabschnitt (212) mit einem Krümmungsradius $r_c$ umfasst.

10. Verfahren zur optimierten Anordnung von Drucksensoren (100) gemäß Anspruch 9, wobei $d < l < 20d$.

11. Verfahren zur optimierten Anordnung von Drucksensoren (100) gemäß Anspruch 9, wobei $d < l < 5d$.

12. Verfahren zur optimierten Anordnung von Drucksensoren (100) gemäß Anspruch 9, wobei $r_c \leq d$.

13. Mit Sensoren versehene Vorrichtung (10), die so angeordnet ist, dass sie die Phasen des Schritts eines Fußes eines Benutzers erkennt, umfassend:

- eine einzelne Gruppe $G_k^*$ von Drucksensoren (100), wobei die einzelne Gruppe $G_k^*$ aus $n = 16$ Drucksensoren (100) besteht, die in einem Arbeitsumfang P angeordnet sind und Koordinaten $(x_i, y_i)$ in Bezug auf ein Referenzsystem $S(x, y)$ aufweisen, wobei der Arbeitsumfang P einen geometrischen Mittelpunkt $CG(x_{CG}, y_{CG})$ in dem Referenzsystem $S(x, y)$ definiert, wobei jeder Drucksensor (100) so angeordnet ist, dass er eine Ausgangsspannung $V_i$ als Reaktion auf einen Druck $p_i$, dem er ausgesetzt ist, erzeugt, mit i = 1,..., $n$;
- eine Steuereinheit, die so angeordnet ist, dass sie die Ausgangsspannung $V_i$ von jedem Drucksensor (100) empfängt und einen entsprechenden Kraftwert $F_i = f(V_i)$ verarbeitet;

wobei unter den $n = 16$ Drucksensoren (100):

- eine Anzahl $n_{rear}$ von Drucksensoren (100) eine Koordinate $y_i < y_{CG}$ aufweist, mit $5 < n_{rear} < 9$;
- eine Anzahl $n_{front}$ von Drucksensoren (100) eine Koordinate $y_i > y_{CG}$ aufweist, mit $7 < n_{front} < 11$.

**Revendications**

1. Procédé permettant l'agencement optimisé de capteurs de pression (100) au sein d'un dispositif (10) muni de capteurs agencé pour détecter des événements biomécaniques caractérisant le pas d'un utilisateur, ledit procédé comprenant les étapes de :

   - pré-agencement d'un dispositif non optimisé (10') muni de capteurs comprenant :
   - un groupe G de $m$ capteurs de pression (100) agencés dans un périmètre de travail P et comportant des coordonnées $(x_i, y_i)$ par rapport à un système de référence $S(x,y)$ solidaire dudit périmètre de travail P, chaque capteur de pression (100) étant agencé pour produire une tension de sortie $V_i$ en réponse à une pression $p_i$ à laquelle il est soumis, avec $i = 1,...,m$ ;
   - une unité de commande agencée pour recevoir ladite tension de sortie $V_i$ en provenance de chaque capteur de pression (100) et pour traiter une valeur correspondante de force $F_i = f(V_i)$ ;
   - exécution d'un cycle de travail à l'aide dudit dispositif non optimisé (10') muni de capteurs, chaque cycle de travail comprenant au moins un pas comprenant les phases de :
   - position précoce ;
   - propulsion ;
   - vol ;
   - traitement de $m$ valeurs de force $F_i(t)$, en fonction du temps $t$, en partant desdites valeurs de tension de sortie $V_i$ acquises durant ledit cycle de travail ;
   - calcul de la force totale $vGRF(t)$ exprimée sur ledit dispositif non optimisé (10') muni de capteurs par l'équation :

$$vGRF(t) = \sum_{i=1}^{m} F_i(t) \quad F_i(t) = \begin{cases} f(V_i) & V_i \leq V_{thresh} \\ 0 & V_i > V_{thresh} \end{cases}$$

   où $V_{thresh}$ est une valeur seuil prédéfinie ;
   - calcul du centre de pressions $CoP_y(t)$ le long d'un axe y dudit système de référence $S(x,y)$ durant ledit cycle de travail par l'équation :

$$CoP_y(t) = \begin{cases} \dfrac{\sum_{i=1}^{m}(F_i \cdot y_i)}{\sum_{i=1}^{m}(F_i)} & vGRF \geq vGRF_{thresh} \\ NaN & vGRF < vGRF_{thresh} \end{cases}$$

   où $vGPF_{thresh}$ est une valeur seuil prédéfinie et $NaN$ est une valeur non détectable
   - calcul du centre géométrique $CG_y$ le long d'un axe $y$ dudit système de référence $S(x,y)$ par l'équation :

$$CG_y = \frac{y_{max} + y_{min}}{2}$$

   où $y_{max}$ et $y_{min}$ sont respectivement les coordonnées maximale et minimale $y_i$ desdits capteurs de pression (100) ;
   - définition de l'instant $t_{HS}$, c'est-à-dire l'instant de début de ladite première phase de position initiale, en tant qu'instant où $CoP_y(t)$ passe d'une valeur $NaN$ à une valeur définie ;
   - définition de l'instant $t_{FF}$, c'est-à-dire l'instant de début de ladite phase de propulsion, en tant que premier instant où $CoP_y(t) \geq CG_y$ ;
   - définition de l'instant $t_{TO}$, c'est-à-dire l'instant de début de ladite phase de vol, en tant qu'instant où $CoP_y(t)$ passe d'une valeur définie à une valeur $NaN$ ;
   - définition d'un nombre $n < m$ de capteurs de pression (100) souhaité dans ledit dispositif (10) muni de capteurs ;
   - définition d'un nombre $C_{n,m}$ de configurations $G_k$ contenant $n$ capteurs de pression (100) dans ledit groupe G de $m$ capteurs de pression (100) dans ledit périmètre de travail P, avec $k = 1,...,C_{n,m}$, avec :

$$C_{n,m} = \frac{m!}{(m-n)! \, n!}$$

- pour chaque configuration $G_k$, calcul de la force totale $vGRF^k(t)$ exprimée sur ledit dispositif (10) muni de capteurs par l'équation :

$$vGRF^k(t) = \sum_{i=1}^{n} F_i^k(t) \quad F_i^k(t) = \begin{cases} f(V_i) & V_i \leq V_{thresh} \\ 0 & V_i > V_{thresh} \end{cases}$$

- pour chaque configuration $G_k$, calcul du centre de pressions $CoP_y^k(t)$ le long d'un axe y dudit système de référence $S(x,y)$ durant ledit cycle de travail par l'équation :

$$CoP_y^k(t) = \begin{cases} \dfrac{\sum_{i=1}^{n}\left(F_i^k \cdot y_i\right)}{\sum_{i=1}^{n}\left(F_i^k\right)} & vGRF^k \geq vGRF_{thresh} \\ NaN & vGRF^k < vGRF_{thresh} \end{cases}$$

- pour chaque configuration $G_k$, définition de l'instant $t_{HS}^k$ en tant qu'instant où $CoP_y^k(t)$ passe d'une valeur $NaN$ à une valeur définie, définition de l'instant $t_{FF}^k$ en tant que premier instant où $CoP_y^k(t) \geq CG_y$, et définition de l'instant $t_{TO}^k$ en tant qu'instant où $CoP_y^k(t)$ passe d'une valeur définie à une valeur $NaN$;
- pour chaque configuration $G_k$, calcul des distances temporelles :

$$\Delta t_{HS}^k = \left| t_{HS}^k - t_{HS} \right|$$

$$\Delta t_{FF}^k = \left| t_{FF}^k - t_{FF} \right|$$

$$\Delta t_{TO}^k = \left| t_{TO}^k - t_{TO} \right|$$

- sélection d'au moins une configuration $G_k^*$ pour laquelle $\Delta t_{HS}^k \leq HS_{thresh}$ et $\Delta t_{TO}^k \leq TO_{thresh}$, où $HS_{thresh}$ et $TO_{thresh}$ sont des seuils prédéfinis de distance temporelle ;
- obtention dudit dispositif (10) muni de capteurs comprenant $n$ capteurs de pression (100) agencés selon l'une de ladite ou de chaque configuration $G_k^*$.

2. Procédé permettant l'agencement optimisé de capteurs de pression (100), selon la revendication 1, une itération des étapes suivantes étant prévue :

- définition d'un nombre $n < m$ de capteurs de pression (100) ;
- définition d'un nombre $C_{n,m}$ de configurations $G_k$ ;
- pour chaque configuration $G_k$ :
- calcul de la force totale $vGRF^k$ ;
- calcul du centre de pressions $CoP_y^k(t)$ ;
- définition des instants $t_{HS}^k$, $t_{FF}^k$, $t_{TO}^k$ ;
- calcul des distances temporelles $\Delta t_{HS}^k = \left| t_{HS}^k - t_{HS} \right|$,

$$\Delta t_{FF}^k = \left| t_{FF}^k - t_{FF} \right| \text{ et } \Delta t_{TO}^k = \left| t_{TO}^k - t_{TO} \right| ;$$

- sélection des configurations $G_k^*$ pour lesquelles $\Delta t_{HS}^k \leq HS_{thresh}$ et $\Delta t_{TO}^k \leq TO_{thresh}$, où $HS_{thresh}$ et $TO_{thresh}$ sont des seuils prédéfinis de distance temporelle ;

à chaque itération desdites étapes, $n$ prenant une valeur entière décroissante comprise entre $m - 1$ et $n_{min}$, où $n_{min}$ est la valeur minimale de $n$ pour laquelle un nombre de configurations $G_k^*$ supérieur à zéro peuvent être sélectionnées.

**3.** Procédé permettant l'agencement optimisé de capteurs de pression (100), selon les revendications 1 ou 2, dans ladite étape de sélection, des configurations sélectionnées existent $G_k^*$ pour lesquelles $\Delta t_{HS}^k \leq HS_{thresh}$, $\Delta t_{FF}^k \leq FF_{thresh}$, $\Delta t_{TO}^k \leq TO_{thresh}$, où $FF_{thresh}$ est un seuil prédéfini de distance temporelle.

**4.** Procédé permettant l'agencement optimisé de capteurs de pression (100), selon les revendications 1 ou 2, une étape de définition d'une configuration préférée au sein desdites configurations $G_k^*$ étant également prévue, ladite configuration préférée étant la configuration comportant au minimum la valeur :

$$err = \Delta t_{HS}^k + \Delta t_{TO}^k + \frac{\left| \Delta t_{HS}^k - \Delta t_{TO}^k \right|}{2}$$

.

**5.** Procédé permettant l'agencement optimisé de capteurs de pression (100), selon l'une quelconque des revendications précédentes, en aval de ladite étape de sélection desdites configurations $G_k^*$, pour chaque configuration $G_k^*$, les étapes suivantes sont prévues :

- définition d'un nombre $b < n$ de capteurs de pression (100) dont la rupture ou le dysfonctionnement doit être simulé ;
- définition d'un nombre $C_{n-b,n}$ de configurations $G_z$ contenant $n-b$ capteurs de pression (100) au sein du nombre $n$ de capteurs de pression (100) dans ledit périmètre de travail P, avec $z = 1, \ldots, C_{n-b,n}$, avec :

$$C_{n-b,n} = \frac{(n-b)!}{b!\, n!}$$

- pour chaque configuration $G_z$, le calcul de la force totale $vGRF^z(t)$ exprimée sur ledit dispositif (10) muni de capteurs par l'équation :

$$vGRF^z(t) = \sum_{i=1}^{n-b} F_i^z(t) \quad F_i^z(t) = \begin{cases} f(V_i) & V_i \leq V_{thresh} \\ 0 & V_i > V_{thresh} \end{cases}$$

- pour chaque configuration $G_z$, calcul du centre de pressions $CoP_y^z(t)$ le long d'un axe y dudit système de référence $S(x,y)$ durant ledit cycle de travail par l'équation :

$$CoP_y^z(t) = \begin{cases} \dfrac{\sum_{i=1}^{n-b}(F_i^z \cdot y_i)}{\sum_{i=1}^{n-b}(F_i^z)} & vGRF^z \geq vGRF_{thresh} \\ NaN & vGRF^z < vGRF_{thresh} \end{cases}$$

- pour chaque configuration $G_z$, définition de l'instant $t_{HS}^z$ en tant qu'instant où $CoP_y^z(t)$ passe d'une valeur $NaN$ à une valeur définie, définition de l'instant $t_{FF}^z$ en tant qu'instant où $CoP_y^z(t) = CG_y$, et définition de l'instant $t_{TO}^z$ en tant qu'instant où $CoP_y^z(t)$ passe d'une valeur définie à une valeur $NaN$;

- pour chaque configuration $G_z$, calcul des distances temporelles :

$$\Delta t_{HS}^{z} = \left| t_{HS}^{z} - t_{HS}^{k} \right|$$

$$\Delta t_{FF}^{z} = \left| t_{FF}^{z} - t_{FF}^{k} \right|$$

$$\Delta t_{TO}^{z} = \left| t_{TO}^{z} - t_{TO}^{k} \right|$$

- sélection des configurations $G_z^*$ pour lesquelles $\Delta t_{HS}^{z} \leq HS_{thresh}^{z}$ et $\Delta t_{TO}^{z} \leq TO_{thresh}^{z}$, où $HS_{thresh}^{z}$ et $TO_{thresh}^{z}$ sont des seuils prédéfinis de distance temporelle.

6. Procédé permettant l'agencement optimisé de capteurs de pression (100), selon la revendication 5, une itération des étapes suivantes étant prévue :

   - définition du nombre $b < n$ de capteurs de pression (100) ;
   - définition d'un nombre $C_{n-b,n}$ de configurations $G_z$ ;
   - pour chaque configuration $G_z$ :
   - calcul de la force totale $vGRF^z$(t) ;
   - calcul du centre de pressions $CoP_y^z(t)$ ;
   - définition des instants $t_{HS}^{z}$, $t_{FF}^{z}$, $t_{TO}^{z}$ ;
   - calcul des distances temporelles $\Delta t_{HS}^{z} = \left| t_{HS}^{z} - t_{HS}^{k} \right|$,

$$\Delta t_{FF}^{z} = \left| t_{FF}^{z} - t_{FF}^{k} \right| \text{ et } \Delta t_{TO}^{z} = \left| t_{TO}^{z} - t_{TO}^{k} \right| ;$$

   - sélection des configurations $G_z^*$ pour lesquelles $\Delta t_{HS}^{z} \leq HS_{thresh}^{z}$ et $\Delta t_{TO}^{z} \leq TO_{thresh}^{z}$, où $HS_{thresh}^{z}$ et $TO_{thresh}^{z}$ sont des seuils prédéfinis de distance temporelle ;

   à chaque itération desdites étapes, $b$ prenant une valeur entière croissante définie entre 1 et $b_{max}$, où $b_{max}$ est la valeur maximale de $b$ pour laquelle un nombre supérieur à zéro de configurations $G_z^*$ peuvent être sélectionnées.

7. Procédé permettant l'agencement optimisé de capteurs de pression (100), selon l'une quelconque des revendications précédentes, durant ladite étape d'exécution dudit cycle de travail, pour chaque configuration $G_k$, une étape de calcul de la force totale $vGRF^k$(t) et une étape de calcul du centre de pressions $CoP_y^k(t)$ par un dispositif externe existent, afin de comparer les valeurs de $vGRF^k(t)$ et $CoP_y^k(t)$ définies par ledit dispositif (10) muni de capteurs avec les valeurs définies par ledit dispositif externe.

8. Procédé permettant l'agencement optimisé de capteurs de pression (100), selon l'une quelconque des revendications précédentes, ledit cycle de travail comprenant un nombre N > 1 de pas et toutes les valeurs définies étant calculées à chaque pas et modérées les unes par rapport aux autres.

9. Procédé permettant l'agencement optimisé de capteurs de pression (100), selon l'une quelconque des revendications précédentes, une étape de connexion dudit dispositif sensoriel (10) à un circuit imprimé (200) étant également prévue comprenant au moins une piste conductrice (210) comportant une géométrie en serpentin et comprenant au moins deux parties linéaires (211) de longueur $l$ et de largeur $d$ et une partie de connexion arquée (212) comportant un rayon de courbure $r_c$.

**10.** Procédé permettant l'agencement optimisé de capteurs de pression (100), selon la revendication 9, $d < l < 20d$.

**11.** Procédé permettant l'agencement optimisé de capteurs de pression (100), selon la revendication 9, $d < l < 5d$.

**12.** Procédé permettant l'agencement optimisé de capteurs de pression (100), selon la revendication 9, $r_c \leq d$.

**13.** Dispositif (10) muni de capteurs agencé pour détecter les phases du pas d'un utilisateur comprenant :

- un groupe unique $G_k^*$ de capteurs de pression (100), ledit groupe unique $G_k^*$ étant constitué de $n$ = 16 capteurs de pression (100) agencés dans un périmètre de travail P et comportant des coordonnées ($x_i, y_i$) par rapport à un système de référence $S(x,y)$, ledit périmètre de travail P définissant un centre géométrique $CG(x_{CG}, y_{CG})$ dans ledit système de référence $S(x,y)$, chaque capteur de pression (100) étant agencé pour produire une tension de sortie $V_i$ en réponse à une pression $p_i$ à laquelle il est soumis, avec i = 1,...,$n$ ;
- une unité de commande agencée pour recevoir ladite tension de sortie $V_i$ en provenance de chaque capteur de pression (100) et pour traiter une valeur correspondante de force $F_i = f(V_i)$ ; parmi lesdits $n$ = 16 capteurs de pression (100) :
- un nombre $n_{arrière}$ de capteurs de pression (100) comportant une coordonnée $y_i < y_{CG}$, avec 5 < $n_{arrière}$ < 9 ;
- un nombre $n_{avant}$ de capteurs de pression (100) comportant une coordonnée $y_i > y_{CG}$, avec 7 < $n_{avant}$ < 11.

# Fig. 1

```
┌─────────────────────────────────┐
│       NOT OPTIMIZED device      │
└─────────────────────────────────┘
                 │
                 ▼
         ┌──────────────┐        ┌─────────────┐
         │  $F_i = f(V_i)$  │──────▶│ define n<m  │
         └──────────────┘        └─────────────┘
          │                            │
          ▼                            ▼
 ┌──────────────────┐     ┌──────────────────────────────┐
 │  G {m sensors}   │     │  $C_{m,n}$ config. $G_k$ {n sensors}  │
 └──────────────────┘     └──────────────────────────────┘
          │                            │
   i = 1, ... , m              i = 1, ... , n
          │                            │
          ▼                            ▼
 ┌──────────────────┐     ┌──────────────────────────────┐
 │ vGRF(t), $CoP_y(t)$ │     │ $vGRF^k(t)$, $CoP_y^k(t)$       │
 └──────────────────┘     └──────────────────────────────┘
          │                            │
          ▼            +        -      ▼
 ┌──────────────────┐     ◯     ┌──────────────────────────────┐
 │ $t_{HS}$, $t_{FF}$, $t_{TO}$ │──────────│ $t_{HS}^k$, $t_{FF}^k$, $t_{TO}^k$  │
 └──────────────────┘           └──────────────────────────────┘
                      │
                      ▼
          ┌───────────────────────────┐   k = 1, ... , $C_{m,n}$
          │ $\Delta t_{HS}^k$, $r\,t_{FF}^k$, $r\,t_{TO}^k$ │
          └───────────────────────────┘
                      │
                      ▼
          ┌───────────────────────────┐
          │  $\Delta t_{HS}^k \leq HS_{thresh}$  │
          │           AND             │
          │  $\Delta t_{TO}^k \leq TO_{thresh}$  │
          └───────────────────────────┘
             │                      │
           YES                     NO
             │                      │
             ▼                      ▼
   ┌──────────────────┐    ┌──────────────┐
   │ $G_k^*$ {n sensors} │    │  redefine n  │
   └──────────────────┘    └──────────────┘
             │
             ▼
   ┌──────────────────────┐
   │ OPTIMIZED device(s)  │
   │  having n sensors    │
   └──────────────────────┘
```

# Fig. 2

# Fig. 3

# Fig. 4

# Fig. 5

# Fig. 6

**Fig. 7**

200

**Fig. 7A**

210

# Fig. 8

## Fig. 9A

200'

210'

220'

## Fig. 9B

220'  200'

210'

## Fig. 9C

200

210

220

## Fig. 9D

200

210

220

# Fig. 10

20

200'

101

110

# Fig. 11

20'

21'

200'

100

100

10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2747645 B1


**Non-patent literature cited in the description**

- **SIMONA CREA et al.** A Wireless Flexible Sensorized Insole for Gait Analysis. *SENSORS,* 09 January 2014, vol. 14, 1073-1093